# EUROPÄISCHE PATENTANMELDUNG

(11) **EP 0 761 651 A2**
(43) Veröffentlichungstag der Anmeldung: **12.03.1997**
(21) Anmeldenummer: 96113291.7
(22) Anmeldetag: 20.08.1996
(51) Int. Cl.: C07D 211/82, C07D 211/84, C07D 211/86, C07D 211/90, C07D 401/04, C07D 409/04, C07D 405/04, C07D 413/04, C07D 491/04

(54) **Verfahren zur Herstellung von optisch aktiven ortho-substituierten 4-Aryl-Dihydropyridinen**

(30) Priorität: 01.09.1995 DE 19532320
(71) Anmelder: BAYER AG, 51368 Leverkusen (DE)
(72) Erfinder: Straub, Alexander, Dr., 42113 Wuppertal (DE)

(57) **Zusammenfassung**

Die Erfindung betrifft ein Verfahren zur Herstellung von optisch aktiven ortho-substituierten 4-Aryl- oder Heteroaryl-1,4-dihydropyridinen durch Oxidation und anschließende Reduktion aus ihren entgegengesetzten Enantiomeren.

## Beschreibung

Die vorliegende Erfindung betrifft ein Verfahren zur Herstellung von optisch aktiven ortho-substituierten 4-Aryl- oder Heteroaryl-1,4-dihydropyridinen durch Oxidation und anschließende Reduktion aus ihren entgegengesetzten Enantiomeren.

In 4-Position substituierte 1,4-Dihydropyridine besitzen an C-4 ein chirales Zentrum, wenn ihr Grundkörper unsymmetrisch substituiert ist. Es ist bereits bekannt, daß die beiden daraus resultierenden Enantiomere sich in ihrer pharmakologischen Wirkung grundlegend unterscheiden können (vergl. EP 26 317 A). Bisherige Verfahren zur Herstellung des gewünschten Enantiomers sind auf den Gebrauch chiraler Auxiliare angewiesen. Sie beinhalten entweder enantioselektive Synthesen oder die Racemattrennung über ein durch achirale Synthesen erhaltenes Diastereomeren-Gemisch. Im letzten Fall erhält man stets 50% des ebenfalls aufwendig synthetisierten "falschen" Enantiomers, für das in der Regel keine Verwendung besteht.

Es ist bereits bekannt, daß 4-(ortho-aryl)-substituierte 1,4-Dihydropyridine bezüglich des 4-Arylrestes in zwei rotameren Formen vorkommen können, der synperiplanaren (sp)- und der wesentlich selteneren antiperiplanaren (ap)-Form. Es ist ferner bekannt, daß Biarylsysteme nur dann stabile Atropisomere bilden können, wenn sie nichtsymmetrisch ortho-substituiert sind, und die freie Rotation um ihre Bindungsachse gehindert ist.

Außerdem sind auch Oxidationen von Dihydropyridinen zu Pyridinen und gezielte Reduktionen von Pyridinen bekannt, wobei aber über den stereochemischen Verlauf solcher Reaktionen an den entsprechenden Enantiomeren keine Erkenntnisse vorliegen.

Die Erfindung betrifft ein kombiniertes oxidativ / reduktives Verfahren zur Herstellung von optisch aktiven 1,4-Dihydropyridinen, die in 4-Stellung ein chirales C-Atom haben und mit einem aromatischen Rest substituiert sind, der mindestens einen Substituenten in ortho-Position trägt.

Typische Vertreter, die nach dem erfindungsgemäßen Verfahren hergestellt werden können, sind z.B. optisch aktive Dihydropyridine, deren chirales C-Atom in 4-Position einen ortho-substituierten Arylrest trägt, der allgemeinen Formel (I) 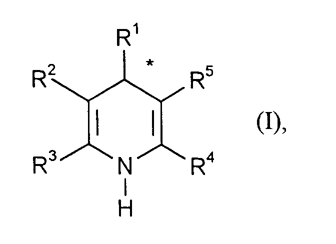 in welcher
- R¹: für einen Arylrest der Formel 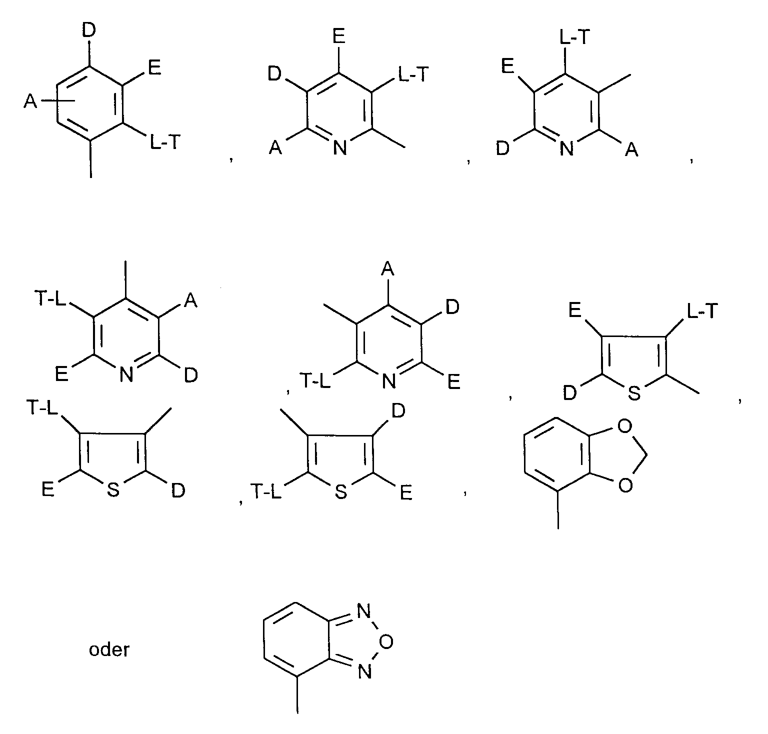 steht,
worin
- A, D und E: gleich oder verschieden sind und
Wasserstoff, Halogen, Cyano, Trifluormethyl oder für geradkettiges oder verzweigtes Alkyl oder Alkenyl mit jeweils bis zu 6 Kohlenstoffatomen bedeuten, die gegebenenfalls durch Aryl mit 6 bis 10 Kohlenstoffatomen, durch einen 5- bis 7-gliedrigen gesättigten oder ungesättigten Heterocyclus mit bis zu 3 Heteroatomen aus der Reihe S, N und/oder O oder durch geradkettiges oder verzweigtes Alkoxycarbonyl mit bis zu 8 Kohlenstoffatomen oder Carboxyl substituiert sind, oder
eine Gruppe der Formel -NR⁶R⁷, -OR⁸, -S(O)ₐR⁹, -SR¹⁰ oder -P(O)(OR¹¹)(OR¹²) bedeuten,
worin
- R⁶, R⁷, R⁸ und R¹⁰: gleich oder verschieden sind und
Wasserstoff, geradkettiges oder verzweigtes Alkyl mit bis zu 8 Kohlenstoffatomen, Benzyl, Aryl mit 6 bis 10 Kohlenstoffatomen oder einen 5- bis 7-gliedrigen gesättigten oder ungesättigten Heterocyclus mit bis zu 3 Heteroatomen aus der Reihe S, N und/oder O bedeuten,
- a: eine Zahl 1 oder 2 bedeutet,
- R⁹: geradkettiges oder verzweigtes Alkyl mit bis zu 6 Kohlenstoffatomen, Benzyl, Phenyl oder Tolyl bedeutet,
- R¹¹ und R¹²: gleich oder verschieden sind und
Wasserstoff oder geradkettiges oder verzweigtes Alkyl mit bis zu 6 Kohlenstoffatomen bedeuten,
- L: eine direkte Bindung, ein Schwefel- oder Sauerstoffatom oder einen Rest der Formel -NH oder SO₂ bedeutet,
- T: in Abhängigkeit von der Definition des Substituenten L eine der chemisch sinnvollen oben angegebenen Bedeutungen von A, D und/oder E hat und mit dieser gleich oder verschieden ist, aber in den Fällen L = Bindung oder -SO₂ nicht für Wasserstoff steht, oder einen Rest der Formel -CHF₂ bedeutet,
oder
- D und E: im direkt benachbarten Fall oder E und T unter Einbezug der aromatischen Doppelbindung jeweils gemeinsam einen 5- bis 8-gliedrigen, partiell ungesättigte oder ungesättigten Carbocyclus oder Heterocyclus mit bis zu 3 Heteroatomen aus der Reihe S, N und/oder O bilden, wobei die Ringsysteme gegebenenfalls bis zu 2-fach gleich oder verschieden durch Halogen, Hydroxy, Nitro, Cyano, Trifluormethyl, durch geradkettiges oder verzweigtes Alkyl oder Alkoxy mit jeweils bis zu 6 Kohlenstoffatomen, durch Aryl mit 6 bis 10 Kohlenstoffatomen oder durch einen 5- bis 6-gliedriegen aromatischen Heterocyclus mit bis zu 3 Heteroatomen aus der Reihe S, N und/oder O substituiert sind,
- R² und R⁵: gleich oder verschieden sind und
für Cyano, Nitro, Carboxyl oder für geradkettiges oder verzweigtes Alkoxycarbonyl mit bis zu 6 Kohlenstoffatomen stehen, das gegebenenfalls durch geradkettiges oder verzweigtes Alkoxy oder Acyl mit jeweils bis zu 8 Kohlenstoffatomen oder durch geradkettiges oder verzweigtes Alkyl mit bis zu 6 Kohlenstoffatomen substituiert ist, das seinerseits durch geradkettiges oder verzweigtes Acyl mit bis zu 4 Kohlenstoffatomen, Pyridyl, Furanyl oder durch eine Gruppe der Formel -NR¹³R¹⁴ substituiert sein kann,
worin
- R¹³ und R¹⁴: gleich oder verschieden sind und
Wasserstoff, geradkettiges oder verzweigtes Alkyl mit bis zu 6 Kohlenstoffatomen, Aryl mit 6 bis 10 Kohlenstoffatomen oder Benzyl bedeuten, wobei die Ringsysteme gegebenenfalls durch Halogen substituiert sind,
oder
Alkoxycarbonyl gegebenenfalls durch eine Gruppe der Formel -NR¹⁵R¹⁶ substituiert ist,
worin
- R¹⁵ und R¹⁶: die oben aufgeführte Bedeutung von R¹³ und R¹⁴ haben und mit dieser gleich oder verschieden sind,
oder
- R² und/oder R⁵: für einen Rest der Formel -PO(OR¹⁷)(OR¹⁸), -SO₂ R¹⁹ oder -CO-NR²⁰R²¹ stehen,
worin
- R¹⁷ und R¹⁸: die oben angegebene Bedeutung von R¹⁰ und R¹¹ haben und mit diesen gleich oder verschieden sind,
oder
- R¹⁷ und R¹⁸: gemeinsam einen Rest der Formel 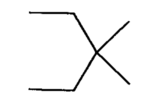 bilden,
- R¹⁹: die oben angegebene Bedeutung von R⁹ hat und mit dieser gleich oder verschieden ist,
- R²⁰ und R²¹: gleich oder verschieden sind und
Wasserstoff, geradkettiges oder verzweigtes Alkyl mit bis zu 6 Kohlenstoffatomen oder Phenyl bedeuten,
- R³ und R⁴: gleich oder verschieden sind und
für Amino, Cyano oder für geradkettiges oder verzweigtes Alkinyl oder Alkyl mit jeweils bis zu 4 Kohlenstoffatomen stehen, das gegebenenfalls durch Hydroxy oder durch eine Gruppe der Formel -(O-CO)_{b}-NR²²R²³ substituiert ist,
worin
- b: eine Zahl 0 oder 1 bedeutet,
- R²² und R²³: gleich oder verschieden sind und die oben angegebene Bedeutung von R⁶ und R⁷ haben,
oder
Alkyl gegebenenfalls durch geradkettiges oder verzweigtes Alkoxy mit bis zu 6 Kohlenstoffatomen substituiert ist, das seinerseits durch eine Gruppe der Formel -NR²⁴R²⁵ substituiert sein kann,
worin
- R²⁴ und R²⁵: die oben angegebene Bedeutung von R¹³ und R¹⁴ haben und mit dieser gleich oder verschieden sind,
oder
- R² und R³ oder R⁴ und R⁵: gemeinsam einen Rest der Formel 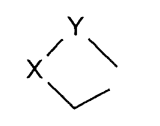 bilden,
worin
- X: ein Sauerstoff- oder ein Schwefelatom oder eine Gruppe der Formel -NR²⁶ oder -(CH₂)_{c} bedeutet,
worin
- R²⁶: Wasserstoff, Phenyl oder geradkettiges oder verzweigtes Alkyl mit bis zu 4 Kohlenstoffatomen bedeutet,
- c: eine Zahl 1, 2 oder 3 bedeutet,
und
- Y: eine Gruppe der Formel CO-, SO₂- oder -SO bedeutet.

Das erfindungsgemäße Verfahren zur Herstellung von optisch aktiven 1,4-Dihydropyridinen ist dadurch gekennzeichnet, daß man 1,4-Dihydropyridine, die in 4-Position ein chirales C-Atom besitzen und durch einen ortho-substituierten Aryl- oder Heteroarylrest substituiert sind, zunächst durch Oxidation in atropisomere Pyridine überführt und diese dann in einem zweiten Schritt wieder zu 1,4-Dihydropyridinen reduziert, wobei entweder eine Inversion zum Spiegelbildisomeren oder unter teilweiser Retention in unterschiedlichem Ausmaß eine Racemisierung stattfindet.

Ein bevorzugtes Verfahren ist darin zu sehen, daß man optisch aktive 1,4-Dihydropyridine, die in 4-Position ein chirales C-Atom besitzen und durch einen ortho-substituierten Arylrest substituiert sind, der Formel (I), die in der synperiplanaren (sp) Form (Ia) oder antiperiplanaren (ap) Form (Ib) bzw. deren Spiegelbildern vorliegen, 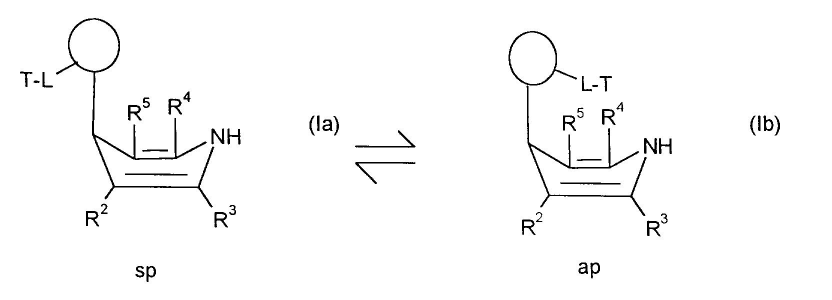 in welchen
- R², R³, R⁴, R⁵, L und T: die oben angegebene Bedeutung haben
und 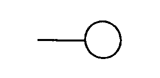 die oben unter dem Substituenten R¹ aufgeführten ortho-substituierten aromatischen Ringsysteme umfaßt,
zunächst durch Oxidation in die atropisomeren Pyridine der allgemeinen Formeln (IIa) bzw. (IIb) 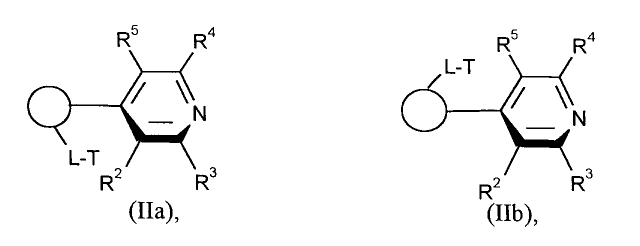 in welchen
R², R³, R⁴, R⁵, L, T und 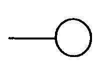 die oben angegebene Bedeutung haben,
überführt und in einem zweiten Schritt diese Pyridine wieder zu 1,4-Dihydropyridinen der Formel (I) reduziert, wobei entweder eine Inversion zum Spiegelbildisomeren oder unter teilweiser Retention eine Racemisierung in unterschiedlichem Ausmaß stattfindet.

Das erfindungsgemäße Verfahren kann durch folgendes Formelschema beispielhaft erläutert werden:

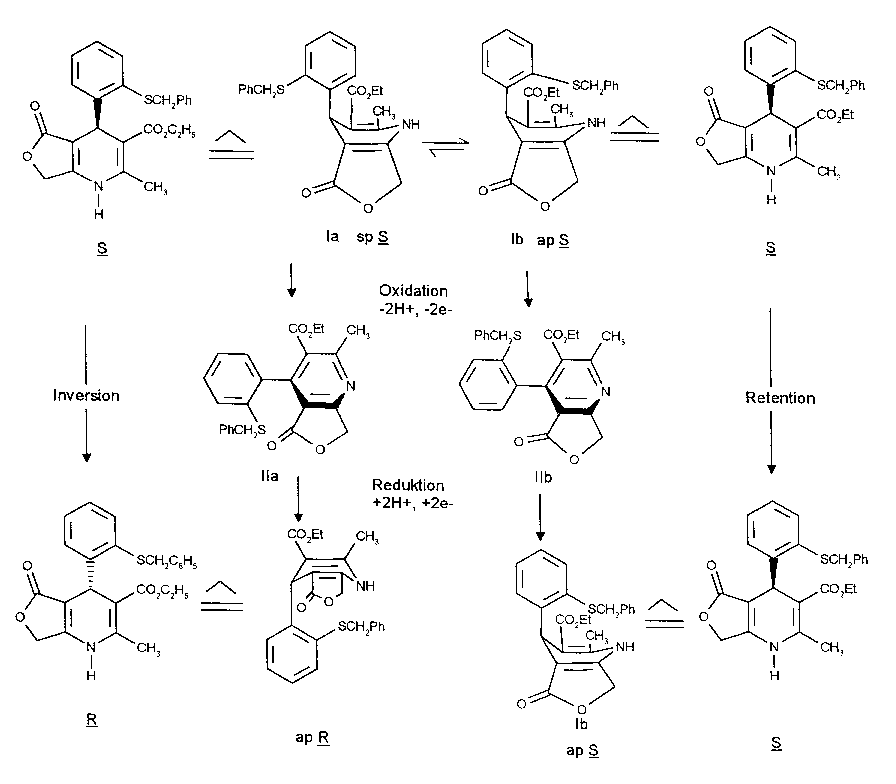

Überraschenderweise erhält man bei der Durchführung des erfindungsgemäßen Verfahrens ausgehend von den unerwünschten 4-ortho-substituierten Aryl- oder Heteroaryl-1,4-dihydropyridin-Enantiomeren deren gewünschte Spiegelbildenantiomere ohne jedes chirales Hilfsauxiliar auf elegante Weise und mit sehr hoher Enantiomerenreinheit und Ausbeute. Darüberhinaus ist es in anderen Fällen möglich, das unerwünschte Enantiomer zu racemisieren, und so die Hälfte des gewünschten Enantiomers zu gewinnen.

Ein weiterer Vorteil des erfindungsgemäßen Verfahrens liegt insbesondere auch im Hinblick auf den Kostenfaktor darin, daß im Gegensatz zu den vielstufigen und oft sehr teuren enantioselektiven chemischen Synthesen oder Trennungsmethoden die gesamte Reaktionssequenz sehr kurz und wenig aufwendig ist.

Überraschenderweise wurde außerdem gefunden, daß man durch geeignete Wahl des Oxidationsmittels eine Veränderung des Atropisomerenverhältnisses eine Bevorzugung des für eine Invertierung notwendigen, aus dem sp-rotameren 1,4-DHP erzeugten, atropisomeren Pyridins erhält.

Weiterhin wurde gefunden, daß die Reduktion der atropisomeren Pyridine mit hoher Enantioselektivität zu den entsprechenden 4-ortho-substituierten Aryl- oder Heteroaryl-1,4-Dihydropyridinen abläuft.

Heterocyclus steht im Rahmen der Erfindung im allgemeinen für einen gesättigten oder ungesättigten 5- bis 8-gliedrigen, vorzugsweise 5- bis 6-gliedrigen Heterocyclus der bis zu 3 Heteroatome aus der Reihe S, N und/oder O enthalten kann. Beispielsweise seien genannt: Pyridyl, Thienyl, Furyl, Pyrrolyl, Thiazolyl, Oxazolyl, Imidazolyl, Morpholinyl oder Piperidyl. Bevorzugt sind Pyridyl und Thienyl.

Als Oxidationsmittel eignen sich in Abhängigkeit von den umzusetzenden 1,4-Dihydropyridinen heterogene (auch unter Verwendung von Trägermaterialien, wie Montmorillonit) und homogene Systeme, anodische Oxidationen, enzymatische Oxidationen z.B. durch P450-Oxidase und Catalase, S₈, NOₓ (x = 1, 2), CrO₃, O₂, KMNO₄/HAc, Pd/C, Chloranil, DDQ, p-Nitrosodimethylanilin, H₂O₂, AgNO₃, Diisoamyldisulfid, Pt/HAc, HgAc₂, I₂, Iodosobenzol, Eisen- oder Nickelcarbonyle, PbO₂, Benzochinon, NaNO₂ in sauren Lösungsmitteln, HNO₃, HNO₂, Fe(NO₃)₃, Cu(NO₃)₂, Br₂/NaOAc, KMnO₄, Ba(MnO₄)₂, MnO₂, DBU, HClO₄, Nitrobenzol, K₃[Fe(CN)₆]/K₂CO₃, Methylacridiniumsalze, Cerammoniumnitrat, Nitrosoniumsalze wie NOBF₄, Nitroniumsalze wie NO₂BF₄, Persäuren wie m-Chlorperbenzoesäure, NaIO₄, Oxone oder Dimethyldioxiran. Bevorzugt sind NO, HNO₃, NOBF₄, NO₂ BF₄ und anodische Oxidationen.

Als Lösemittel für die Oxidationen eignen sich im allgemeinen inerte Lösemittel, die sich unter den Reaktionsbedingungen nicht verändern. Dazu gehören beispielsweise Acetonitril, Salzsäure, Essigsäure, Schwefelsäure, Salpetersäure, DMF, Wasser, Benzol, DMSO, Chloranilin, Aceton, Eisessig, Ether wie beispielsweise Dioxan, Diethylether, Diisopropylether oder Tetrahydrofuran oder chlorierte Kohlenwasserstoffe wie beispielsweise Dichlormethan, Chloroform oder Tetrachlorkohlenstoff. Ebenso ist es möglich, Gemische der oben aufgeführten Lösemittel und/oder Wasser einzusetzen. Bevorzugt sind Chloroform, Eisessig, Aceton, Dioxan, Acetonitril, Dichlormethan oder Tetrachlorkohlenstoff.

Die Reaktionstemperaturen können in einem größeren Bereich variiert werden. Im allgemeinen arbeitet man zwischen -60°C und +200°C, vorzugsweise zwischen 0°C und +100°C.

Gegebenenfalls wird die Oxidation auch unter Schutzgasatmosphäre durchgeführt.

Die Reduktionen werden im allgemeinen durch Reduktionsmittel wie beispielsweise NaBH₄, BH₃, LiAlH₄, Natriumdithionit oder durch kathodische Reduktionen in einer üblichen Elektrolysezelle, bestehend aus Kathode, Anode und Referenzelektrode in Lösung, gegebenenfalls unter Zugabe eines Leitelektrolyten, durchgeführt. Die Zelle kann durch ein Diaphragma in Kathoden- und Anodenraum geteilt oder ungeteilt sein, wobei gleichzeitig die oben beschriebene Oxidation des DHP an der Anode ablaufen kann.

Als Kathodenmaterialien eignen sich im allgemeinen die in der Elektrochemie üblichen Materialien. Bevorzugt sind die Quecksilberpoolelektrode, Graphit, Kohle, Pt, Pd, Pb, Cd oder Ni.

Als Anodenmaterialien eignen sich ebenfalls die üblichen Materialien, wobei Pd, Kohle oder Graphit bevorzugt sind.

Als Leitsalze werden im Rahmen der Erfindung quartäre Ammonium-, Alkali- und Erdalkalisalze eingesetzt. Bevorzugt sind die entsprechenden Halogenide, Perchlorate, Tetrafluorborate, Hexafluorplatinate, Sulfate oder Phosphate.

Als Lösemittel eignen sich im allgemeinen protische und aprotische Solventien und deren Gemische wie beispielsweise Acetonitril, HMPT, DMSO, DMF, Wasser, Pufferlösungen aus Phosphat-, Acetat- oder Citratsalzen, Dimethylacetamid, N-Methylpyrrolidon, Sulfolan, Dichlormethan und Aceton.

Die kathodische Reduktion wird im allgemeinen spannungskontrolliert zwischen -0,5 V und -2,5 V, bevorzugt -1 V und -1,9 V oder stromkontrolliert mit Stromstärken von 5 mA bis 1 A, bevorzugt 10 mA bis 500 mA durchgeführt. Sie kann gegebenenfalls unter Schutzgasatmosphäre durchgeführt werden.

Bevorzugt werden nach dem erfindungsgemäßen Verfahren Dihydropyridine der allgemeinen Formel (I),
in welcher
- R¹: für einen Rest der Formel 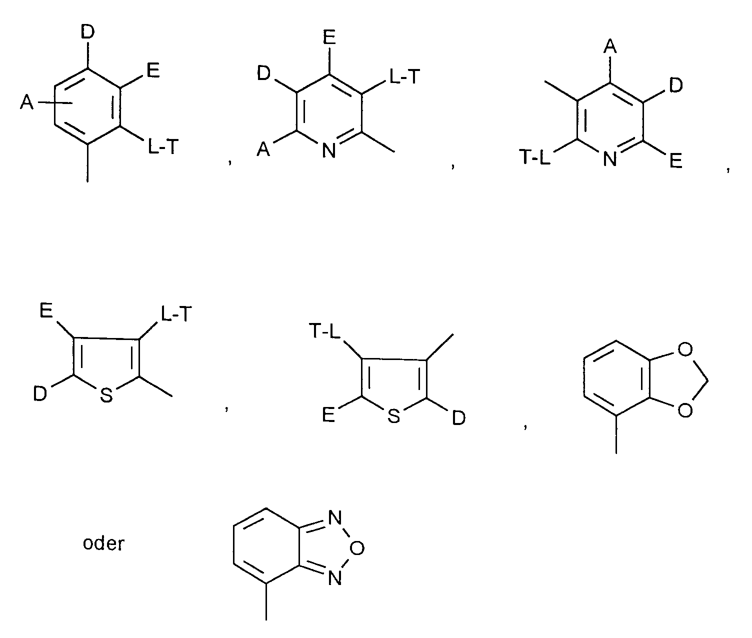 steht,
worin
- A, D und E: gleich oder verschieden sind und
Wasserstoff, Fluor, Chlor, Brom, Cyano, Trifluormethyl oder geradkettiges oder verzweigtes Alkyl oder Alkenyl mit jeweils bis zu 5 Kohlenstoffatomen bedeuten, die gegebenenfalls durch Phenyl, Naphthyl, Pyridyl oder durch geradkettiges oder verzweigtes Alkoxycarbonyl mit bis zu 6 Kohlenstoffatomen oder Carboxyl substituiert sind, oder
eine Gruppe der Formel -NR⁶R⁷, -OR⁸, -S(O)ₐR⁹, -SR¹⁰ oder -P(O)(OR¹¹)(OR¹²) bedeuten,
worin
- R⁶, R⁷, R⁸ und R¹⁰: gleich oder verschieden sind und
Wasserstoff, geradkettiges oder verzweigtes Alkyl mit bis zu 6 Kohlenstoffatomen, Benzyl, Phenyl, Pyridyl, Furyl oder Thienyl bedeuten,
- a: eine Zahl 1 oder 2 bedeutet,
- R⁹: geradkettiges oder verzweigtes Alkyl mit bis zu 4 Kohlenstoffatomen, Benzyl, Phenyl oder Tolyl bedeutet,
- R¹¹ und R¹²: gleich oder verschieden sind und
Wasserstoff oder geradkettiges oder verzweigtes Alkyl mit bis zu 3 Kohlenstoffatomen bedeuten,
- L: eine direkte Bindung, ein Schwefel- oder Sauerstoffatom oder einen Rest der Formel -NH oder SO₂ bedeutet,
- T: in Abhängigkeit von der Definition des Substituenten L eine der chemisch sinnvollen oben angegebenen Bedeutungen von A, D und/oder E hat und mit dieser gleich oder verschieden ist, aber in den Fällen L = Bindung oder -SO₂ nicht für Wasserstoff steht, oder einen Rest der Formel -CHF₂ bedeutet,
oder
- D und E: im direkt benachbarten Fall oder E und T unter Einbezug der aromatischen Doppelbindung jeweils gemeinsam einen ankondensierten Cyclopentenyl-, Cyclohexenyl-, Cycloheptenyl, Cyclohexadienyl-, Cycloheptadienyl- oder Pyridylring bilden, wobei die Ringsysteme gegebenenfalls durch Phenyl, Thienyl, geradkettiges oder verzweigtes Alkyl mit bis zu 4 Kohlenstoffatomen oder Pyridyl substituiert sind,
- R² und R⁵: gleich oder verschieden sind und für Cyano, Nitro, Carboxyl oder für geradkettiges oder verzweigtes Alkoxycarbonyl mit bis zu 5 Kohlenstoffatomen stehen, das gegebenenfalls durch geradkettiges oder verzweigtes Alkoxy oder Acyl mit jeweils bis zu 6 Kohlenstoffatomen oder durch geradkettiges oder verzweigtes Alkyl mit bis zu 5 Kohlenstoffatomen substituiert ist, das seinerseits durch geradkettiges oder verzweigtes Acyl mit bis zu 3 Kohlenstoffatomen, Pyridyl, Furanyl oder durch eine Gruppe der Formel -NR¹³R¹⁴ substituiert sein kann,
worin
- R¹³ und R¹⁴: gleich oder verschieden sind und Wasserstoff, geradkettiges oder verzweigtes Alkyl mit bis zu 5 Kohlenstoffatomen, Phenyl, Naphthyl oder Benzyl bedeuten, wobei die Ringsysteme gegebenenfalls durch Fluor, Chlor oder Brom substituiert sind
oder
Alkoxycarbonyl gegebenenfalls durch eine Gruppe der Formel -NR¹⁵R¹⁶ substituiert ist
worin
- R¹⁵ und R¹⁶: die oben aufgeführte Bedeutung von R¹³ und R¹⁴ haben und mit dieser gleich oder verschieden sind,
oder
- R² und/oder R⁵: für einen Rest der Formel -PO(OR¹⁷)(OR¹⁸), -SO₂ R¹⁹ oder -CO-NR²⁰R²¹ stehen,
worin
- R¹⁷ und R¹⁸: die oben angegebene Bedeutung von R¹⁰ und R¹¹ haben und mit diesen gleich oder verschieden sind,
oder
- R¹⁷ und R¹⁸: gemeinsam einen Rest der Formel 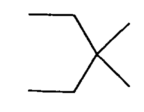 bilden,
- R¹⁹: die oben angegebene Bedeutung von R⁹ hat und mit dieser gleich oder verschieden ist,
- R²⁰ und R²¹: gleich oder verschieden sind und Wasserstoff, geradkettiges oder verzweigtes Alkyl mit bis zu 4 Kohlenstoffatomen oder Phenyl bedeuten,
- R³ und R⁴: gleich oder verschieden sind und für Amino, Cyano oder für geradkettiges oder verzweigtes Alkinyl oder Alkyl mit jeweils bis zu 3 Kohlenstoffatomen stehen, das gegebenenfalls durch Hydroxy oder durch eine Gruppe der Formel -(O-CO)_{b}-NR²²R²³ substituiert ist,
worin
- b: eine Zahl 0 oder 1 bedeutet,
- R²² und R²³: gleich oder verschieden sind und die oben angegebene Bedeutung von R⁶ und R⁷ haben,
oder
Alkyl gegebenenfalls durch geradkettiges oder verzweigtes Alkoxy mit bis zu 4 Kohlenstoffatomen substituiert ist, das seinerseits durch eine Gruppe der Formel -NR²⁴R²⁵ substituiert sein kann,
worin
- R²⁴ und R²⁵: die oben angegebene Bedeutung von R¹³ und R¹⁴ haben und mit dieser gleich oder verschieden sind,
oder
- R² und R³ oder R⁴ und R⁵: gemeinsam einen Rest der Formel 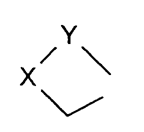 bilden,
worin
- X: ein Sauerstoff- oder ein Schwefelatom oder eine Gruppe der Formel -NR²⁶ oder -(CH₂)_{c} bedeutet,
worin
- R²⁶: Wasserstoff, Phenyl oder geradkettiges oder verzweigtes Alkyl mit bis zu 3 Kohlenstoffatomen bedeutet,
- c: eine Zahl 1, 2 oder 3 bedeutet,
und
- Y: eine Gruppe der Formel CO-, SO₂- oder -SO bedeutet,
hergestellt.

Besonders bevorzugt werden nach dem erfindungsgemäßen Verfahren Verbindungen der allgemeinen Formel (I),
in welcher
- R¹: für einen Rest der Formel 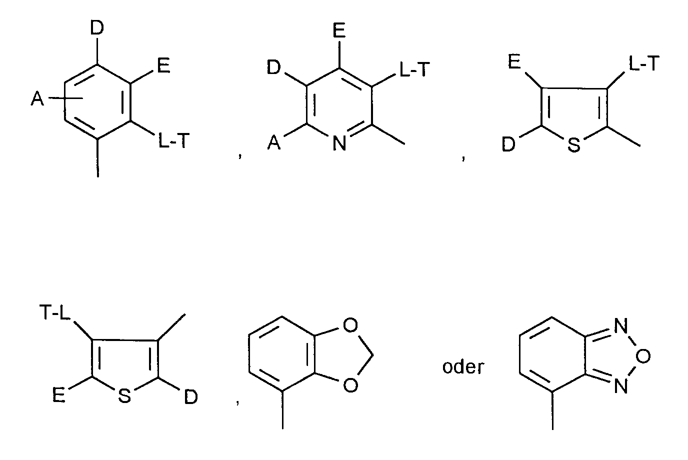 steht,
worin
- A, D und E: gleich oder verschieden sind und
Wasserstoff, Fluor, Chlor, Brom, Cyano, Trifluormethyl oder geradkettiges oder verzweigtes Alkyl oder Alkenyl mit jeweils bis zu 4 Kohlenstoffatomen bedeuten, die gegebenenfalls durch Phenyl, Naphthyl, Pyridyl oder durch geradkettiges oder verzweigtes Alkoxycarbonyl mit bis zu 5 Kohlenstoffatomen oder Carboxyl substituiert sind, oder
eine Gruppe der Formel -NR⁶R⁷, -OR⁸, -S(O)ₐR⁹, -SR¹⁰ oder -P(O)(OR¹¹)(OR¹²) bedeuten,
worin
- R⁶, R⁷, R⁸ oder R¹⁰: gleich oder verschieden sind und Wasserstoff, geradkettiges oder verzweigtes Alkyl mit bis zu 4 Kohlenstoffatomen, Benzyl, Phenyl, Pyridyl oder Furyl bedeuten,
- a: eine Zahl 1 oder 2 bedeutet,
- R⁹: Methyl, Benzyl, Phenyl oder Tolyl bedeutet,
- R¹¹ und R¹²: gleich oder verschieden sind und
Wasserstoff oder geradkettiges oder verzweigtes Alkyl mit bis zu 3 Kohlenstoffatomen bedeuten,
- L: eine direkte Bindung, ein Schwefel- oder Sauerstoffatom oder einen Rest der Formel -NH oder SO₂ bedeutet,
- T: in Abhängigkeit von der Definition des Substituenten L eine der chemisch sinnvollen oben angegebenen Bedeutungen von A, D und/oder E hat und mit dieser gleich oder verschieden ist, aber in den Fällen L = Bindung oder -SO₂ nicht für Wasserstoff steht, oder einen Rest der Formel -CHF₂ bedeutet,
oder
- D und E: im direkt benachbarten Fall oder E und T unter Einbezug der aromatischen Doppelbindung jeweils gemeinsam einen ankondensierten Cyclopentenyl-, Cyclohexenyl-, Cycloheptenyl, Cyclohexadienyl-, Cycloheptadienyl- oder Pyridylring bilden, wobei die Ringsysteme gegebenenfalls durch Phenyl, Thienyl, geradkettiges oder verzweigtes Alkyl mit bis zu 3 Kohlenstoffatomen oder Pyridyl substituiert sind,
- R² und R⁵: gleich oder verschieden sind und Cyano, Nitro oder Carboxyl oder geradkettiges oder verzweigtes Alkoxycarbonyl mit bis zu 4 Kohlenstoffatomen bedeuten, das gegebenenfalls durch geradkettiges oder verzweigtes Alkoxy oder Acyl mit jeweils bis zu 3 Kohlenstoffatomen oder durch geradkettiges oder verzweigtes Alkyl mit bis zu 3 Kohlenstoffatomen substituiert ist, das seinerseits durch geradkettiges oder verzweigtes Acyl mit bis zu 3 Kohlenstoffatomen, Pyridyl, Furanyl oder durch eine Gruppe der Formel -NR¹³R¹⁴ substituiert sein kann,
worin
- R¹³ und R¹⁴: gleich oder verschieden sind und Wasserstoff oder geradkettiges oder verzweigtes Alkyl mit bis zu 4 Kohlenstoffatomen bedeuten,
oder
- R² und/oder R⁵: für einen Rest der Formel -PO(OR¹⁷)(OR¹⁸), -SO₂ R¹⁹ oder -CO-NR²⁰R²¹ stehen,
worin
- R¹⁷ und R¹⁸: die oben angegebene Bedeutung von R¹⁰ und R¹¹ haben und mit diesen gleich oder verschieden sind,
oder
- R¹⁷ und R¹⁸: gemeinsam einen Rest der Formel 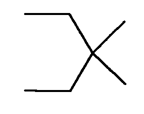 bilden,
- R¹⁹: die oben angegebene Bedeutung von R⁹ hat und mit dieser gleich oder verschieden ist,
- R²⁰ und R²¹: gleich oder verschieden sind und Wasserstoff oder geradkettiges oder verzweigtes Alkyl mit bis zu 3 Kohlenstoffatomen bedeuten,
- R³ und R⁴: gleich oder verschieden sind und für Amino, Cyano oder für geradkettiges oder verzweigtes Alkinyl oder Alkyl mit jeweils bis zu 3 Kohlenstoffatomen stehen, das gegebenenfalls durch Hydroxy oder durch eine Gruppe der Formel -(O-CO)_{b}-NR²²R²³ substituiert ist,
worin
- b: eine Zahl 0 oder 1 bedeutet,
- R²² und R²³: gleich oder verschieden sind und die oben angegebene Bedeutung von R⁶ und R⁷ haben,
oder
Alkyl gegebenenfalls durch geradkettiges oder verzweigtes Alkoxy mit bis zu 3 Kohlenstoffatomen substituiert ist, das seinerseits durch eine Gruppe der Formel -NR²⁴R²⁵ substituiert sein kann,
worin
- R²⁴ und R²⁵: die oben angegebene Bedeutung von R¹³ und R¹⁴ haben und mit dieser gleich oder verschieden sind,
oder
- R² und R³ oder R⁴ und R⁵: gemeinsam einen Rest der Formel 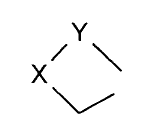 bilden,
worin
- X: ein Sauerstoff- oder ein Schwefelatom oder eine Gruppe der Formel -NR²⁶ oder -(CH₂)_{c} bedeutet,
worin
- R²⁶: Wasserstoff, Phenyl oder geradkettiges oder verzweigtes Alkyl mit bis zu 3 Kohlenstoffatomen bedeutet,
- c: eine Zahl 1, 2 oder 3 bedeutet,
und
- Y: die CO-Gruppe bedeutet,
hergestellt.

Das erfindungsgemäße Verfahren ermöglicht auf elegante Weise die Überführung unerwünschter 4-ortho-substituierter Aryl- oder Heteroaryl-1,4-Dihydropyridin-Enantiomere in die entsprechenden spiegelbildlichen Enantiomeren der allgemeinen Formel (I), die wertvolle Arzneimittel darstellen.

### Experimenteller Teil

Die präparative Elektrochemie wurde mit einem Stanford Wenking Potentiostat ST 72 durchgeführt. Als Reaktionsgefäß diente eine durch Diaphragma in Anoden- und Kathodenraum geteilte Elektrolysezelle. Für Reduktionen wurde eine Quecksilberpoolelektrode verwendet. Das eingesetzte Quecksilber wurde nach jeder Reaktion im Schütteltrichter mit Methanol gewaschen und wiederverwendet. Als Referenzelektrode diente eine gesättigte Kalomelelektrode, die über eine Luggin-Kapillare an den Kathodenraum gekoppelt war. Als Anode bzw. bei Oxidationen auch als Kathode, diente eine Platinblechelektrode. Die Zelle wurde bei 20 °C thermostatisiert und in einer Argonatmosphäre betrieben. Zur Oxidation der DHP's wurde "Mangandioxid gefällt aktiv, zur Synthese", Art.-Nr. 805958 von Merck Schuchardt verwendet.

### Beispiel 1

### Oxidation von (-)-Ethyl-2-methyl-4-(2-benzylthiophenyl)-5-oxo-1,4,5,7-tetrahydrofuro[3,4-b]pyridin-3-carboxylat zu (+)-Ethyl-2-methyl-4-(2-benzylthiophenyl)-5-oxo-5,7-dihydrofuro[3,4-b]pyridin-3-carboxylat mit MnO₂

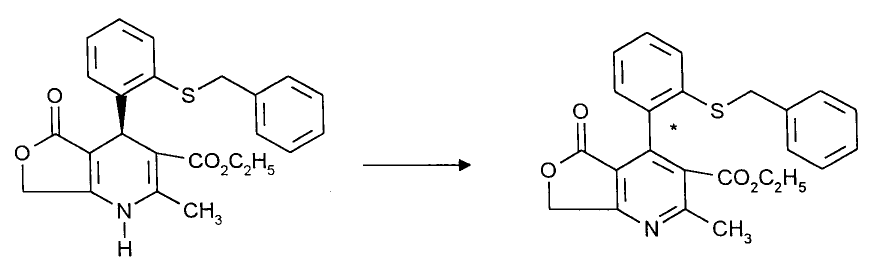

1.37 g (3.3 mmol) (-)-Ethyl-2-methyl-4-(2-benzylthiophenyl)-5-oxo-1,4,5,7-tetrahydrofuro[3,4-b]pyridin-3-carboxylat werden in 150 ml Chloroform gelöst und nach Zugabe von 5.73 g MnO₂ im Ultraschallbad beschallt. Nach Absaugen über Kieselgur und Verdampfen des Lösungsmittels im Vakuum erhält man 1.2 g (87 % d. Th.) (+)-Ethyl-2-methyl-4-(2-benzylthiophenyl)-5-oxo-5,7-dihydrofuro[3,4-b]pyridin-3-carboxylat
[α]^{**20**}_{**D**} = 51.35 ° (c = 0,56, CHCl₃)
HPLC: Atropisomer 2 in der Elutionsfolge
MS (EI): 419 (M⁺, 15 %), 91(100 %)

### Beispiel 2

### Oxidation von (-)-Ethyl-2-methyl-4-(2-benzylthiophenyl)-5-oxo-1,4,5,7-tetrahydrofuro[3,4-b]pyridin-3-carboxylat mit K₃(Fe(CN)₆)

50 mg (0.12 mmol) (-)-Ethyl-2-methyl-4-(2-benzylthiophenyl)-5-oxo-1,4,5,7-tetrahydrofuro[3,4-b]pyridin-3-carboxylat werden in 3 ml Acetonitril mit 1 g K₃(Fe(CN)₆) in 4 ml Wasser versetzt und nach Zugabe einer Spatelspitze K₂CO₃ über Nacht bei RT unter einer Argonatmosphäre gerührt. Nach Zugabe von 1 g K₃(Fe(CN)₆) und 200 mg K₂CO₃ wird anschließend noch 1 h gerührt, anschließend in 50 ml Wasser gegeben, mit Essigester extrahiert und auf Kieselgel (Toluol → Toluol / Essigester = 5 : 1) chromatographiert.
Ausbeute: 4 mg (8 % d. Th.)

### Beispiel 3

### Oxidation von (-)-Ethyl-2-methyl-4-(2-benzylthiophenyl)-5-oxo-1,4,5,7-tetrahydrofuro[3,4-b]pyridin-3-carboxylat mit Cer(IV)ammoniumnitrat

Zu 40 mg (0.1 mmol) (-)-Ethyl-2-methyl-4-(2-benzylthiophenyl)-5-oxo-1,4,5,7-tetrahydrofuro[3,4-b]pyridin-3-carboxylat in 8 ml Aceton wird unter einer Argonatmosphäre eine Lösung von 0.15 g Cer(IV)ammoniumnitrat in 1 ml Wasser getropft. Man rührt 3 h bei RT, gibt in Wasser, extrahiert mit Essigester und bestimmt den ee des nach Abziehen des Lösungsmittels im Vakuum verbleibenden Rückstand mittels chiralem HPLC.

### Beispiel 4

### Oxidation von (-)-Ethyl-2-methyl-4-(2-benzylthiophenyl)-5-oxo-1,4,5,7-tetrahydrofuro[3,4-b]pyridin-3-carboxylat mit Chloranil

Eine Mischung aus 40 mg (0.1 mmol) (-)-Ethyl-2-methyl-4-(2-benzylthiophenyl)-5-oxo-1,4,5,7-tetrahydrofuro[3,4-b]pyridin-3-carboxylat, 21 mg Chloranil und 5 ml Toluol wird über Nacht in einer Argonatmosphäre gekocht. Nach Waschen mit Wasser und Extrahieren mit Essigester bestimmt man den ee.

### Beispiel 5

### Oxidation von (-)-Ethyl-2-methyl-4-(2-benzylthiophenyl)-5-oxo-1,4,5,7-tetrahydrofuro[3,4-b]pyridin-3-carboxylat an der Platinanode

100 mg (0.24 mmol) (-)-Ethyl-2-methyl-4-(2-benzylthiophenyl)-5-oxo-1,4,5,7-tetrahydrofuro[3,4-b]pyridin-3-carboxylat werden mit 0.7 g TEABF₄ als Leitsalz in 35 ml Acetonitril in den Anodenraum einer geteilten Diaphragmazelle gegeben. Der Kathodenraum bestand aus dem substratfreien Elektrolyten. Man elektrolysiert bei ca. 30 mA und kontrolliert dünnschichtchromatographisch die Reaktion, in deren Verlauf ein Zwischenprodukt auftritt. Nach Beendigung wurde in Wasser ausgefällt, abgesaugt und auf Kieselgel chromatographiert.
Ausbeute: 52 mg (52 %).
- R_{f} (SiO₂, Toluol / Essigester = 1:1):: (-)-Ethyl-2-methyl-4-(2-benzylthiophenyl)-5-oxo-1,4,5,7-tetrahydrofuro[3,4-b]pyridin-3-carboxylat: 0,18
(+)-Ethyl-2-methyl-4-(2-benzylthiophenyl)-5-oxo-5,7-dihydrofuro[3,4-b]pyridin-3-carboxylat: 0,54

### Beispiel 6

### Oxidation von (-)-Ethyl-2-methyl-4-(2-benzylthiophenyl)-5-oxo-1,4,5,7-tetrahydrofuro[3,4-b]pyridin-3-carboxylat mit NOBF₄ bzw. NO₂BF₄

20 mg (0.05 mmol) (-)-Ethyl-2-methyl-4-(2-benzylthiophenyl)-5-oxo-1,4,5,7-tetrahydrofuro[3,4-b]pyridin-3-carboxylat werden in 1 ml Acetonitril mit einer Lösung von 6 mg NOBF₄ (oder NO₂BF₄) in 1 ml Acetonitril versetzt. Der Ansatz verfärbt sich leicht gelblich und ist innerhalb 20 min. quantitativ abreagiert. Nach Einrotieren wird mit wäßriger NaHCO₃-Lösung gerührt und mit Essigester extrahiert.

### Beispiel 7

### Oxidation von (-)-Ethyl-2-methyl-4-(2-benzylthiophenyl)-5-oxo-1,4,5,7-tetrahydrofuro[3,4-b]pyridin-3-carboxylat mit konz. HNO₃

100 mg (0.24 mmol) (-)-Ethyl-2-methyl-4-(2-benzylthiophenyl)-5-oxo-1,4,5,7-tetrahydrofuro[3,4-b]pyridin-3-carboxylat werden in 10 ml Chloroform gegeben und nach Zugabe von 3 ml 65proz. HNO₃ heftig gerührt. Nach 2 min wird in 100 ml Wasser gegeben, mit K₂CO₃ neutralisiert, mit Chloroform extrahiert, mit MgSO₄ getrocknet und im Vakuum eingeengt. Nach Chromatographie auf Kieselgel mit Toluol als Eluens erhält man 40 mg (40 % d. Th.) der Pyridinverbindung, dessen ee im chiralen HPLC bestimmt wird.

### Beispiel 8

### Oxidation von (-)-Ethyl-2-methyl-4-(2-benzylthiophenyl)-5-oxo-1,4,5,7-tetrahydrofuro[3,4-b]pyridin-3-carboxylat mit N-Methylacridiniumjodid

Ein Lösung von 300 mg (0.71 mmol) (-)-Ethyl-2-methyl-4-(2-benzylthiophenyl)-5-oxo-1,4,5,7-tetrahydrofuro[3,4-b]pyridin-3-carboxylat, 800 mg (2.49 mmol) N-Methylacridiniumjodid, 150 ml Acetonitril und 1.6 ml 2N H₂SO₄ wird über Nacht in einer Argonatmosphäre unter Rückfluß gekocht. Nach dem Abkühlen wird von evtl. ausgefallenem Niederschlag abgesaugt und die Lösung nach Behandlung mit wässr. NaHCO₃-Lösung mit Essigester extrahiert, eingedampft und chromatographiert.
Ausbeute: 96 mg (32 % d. Th.)

### Beispiel 9

### Oxidation von (-)-Ethyl-2-methyl-4-(2-benzylthiophenyl)-5-oxo-1,4,5,7-tetrahydrofuro[3,4-b]pyridin-3-carboxylat mit NaNO₂/HCl in Ether bzw. CHCl₃

Man überschichtet in einem schmalen Glasgefäß 0.74 g NaNO₂ mit 10 ml Ether und gibt langsam 1 ml halbkonz. HCl hinzu. Von dieser Lösung gibt man umgehend 1 ml der etherischen Phase zu einer Lösung von 36 mg (0.085 mmol) (-)-Ethyl-2-methyl-4-(2-benzylthiophenyl)-5-oxo-1,4,5,7-tetrahydrofuro[3,4-b]pyridin-3-carboxylat in 2 ml Dioxan oder von 30 mg (0.07 mmol) (-)-Ethyl-2-methyl-4-(2-benzyl-thiophenyl)-5-oxo-1,4,5,7-tetrahydrofuro[3,4-b]pyridin-3-carboxylat in 3 ml CHCl₃. Die Reaktionszeiten betragen im ersten Fall 30 min und im zweiten 4 min. Dann wird mit K₂HPO₄-Puffer versetzt, die organische Phase abgetrennt und über eine kleine Säule chromatographiert

### Beispiel 10

### Oxidation von (-)-Ethyl-2-methyl-4-(2-benzylthiophenyl)-5-oxo-1,4,5,7-tetrahydrofuro[3,4-b]pyridin-3-carboxylat mit NaNO₂/HCl in Dioxan

40 mg (0.095 mmol) (-)-Ethyl-2-methyl-4-(2-benzylthiophenyl)-5-oxo-1,4,5,7-tetrahydrofuro[3,4-b]pyridin-3-carboxylat werden in 1 ml Dioxan und 0.56 ml 10 proz. HCl gelöst und mit einer Lösung von 16.8 mg NaNO₂ in 0.1 ml Wasser versetzt, Nach 8 h wird neutralisiert und mit Essigester extrahiert.

### Beispiel 11

### Oxidation von (-)-Ethyl-2-methyl-4-(2-benzylthiophenyl)-5-oxo-1,4,5,7-tetrahydrofuro[3,4-b]pyridin-3-carboxylat mit Cu₄(NO₃)₂ auf Montmorillonit

30 mg (-)-Ethyl-2-methyl-4-(2-benzylthiophenyl)-5-oxo-1,4,5,7-tetrahydrofuro[3,4-b]pyridin-3-carboxylat werden in 0.6 ml Chloroform gelöst und nach Zugabe von 40 mg Cu₄(NO₃)₂ auf Montmorillonit (1.7 mmol/g) von Fluka 8 min ins Ultraschallbad gehalten. Nach Absaugen, Einrotieren und Chromatographieren wird der ee bestimmt.

### Beispiel 12

### Oxidation von (-)-Ethyl-2-methyl-4-(2-benzylthiophenyl)-5-oxo-1,4,5,7-tetrahydrofuro[3,4-b]pyridin-3-carboxylat mit NaNO₂ in Eisessig

0.5 g (-)-Ethyl-2-methyl-4-(2-benzylthiophenyl)-5-oxo-1,4,5,7-tetrahydro-furo[3,4-b]pyridin-3-carboxylat werden in 15 ml Eisessig gelöst. Zu der Lösung, die sich in einem 18 x 180 mm großen Reagenzglas befindet, gibt man innerhalb 10 min 0.5 g NaNO₂, wobei nitrose Gase durch die Lösung perlen. Anschließend wird der Ansatz umgehend in eine Lösung von 25 g K₂HPO₄ in 50 ml Wasser gegeben, gerührt, mit Essigester extrahiert und auf Kieselgel chromatographiert. Man erhält 418 mg (84 % d. Th.) (+)-Ethyl-2-methyl-4-(2-benzylthiophenyl)-5-oxo-5,7-dihydrofuro[3,4-b]pyridin-3-carboxylat.

### Beispiel 13

### Oxidation von (-)-Ethyl-2-methyl-4-(2-benzylthiophenyl)-5-oxo-1,4,5,7-tetrahydrofuro[3,4-b]pyridin-3-carboxylat mit verdünnter HNO₃

100 mg (0.24 mmol) (-)-Ethyl-2-methyl-4-(2-benzylthiophenyl)-5-oxo-1,4,5,7-tetrahydrofuro[3,4-b]pyridin-3-carboxylat werden in 10 ml Chloroform gegeben, mit 4 ml 3N HNO₃ versetzt und unter intensivem Rühren gekocht, bis das DC vollständigen Umsatz zeigt.

### Beispiel 14

### Oxidation von (-)-Ethyl-2-methyl-4-(2-benzylthiophenyl)-5-oxo-1,4,5,7-tetrahydrofuro[3,4-b]pyridin-3-carboxylat mit KMnO₄

40 mg (0.095 mmol) (-)-Ethyl-2-methyl-4-(2-benzylthiophenyl)-5-oxo-1,4,5,7-tetrahydrofuro[3,4-b]pyridin-3-carboxylat, 16 mg KMnO₄ und 35 mg Montmorillonit KSF werden in 0.8 ml CH₂Cl₂ und 0.5 ml Wasser (Version 1) bzw. in 3 ml Benzol (Version 2) aufgenommen. Im ersten Fall ist die Reaktion nach 2-stündigem Beschallen mit Ultraschall beendet, im zweiten Fall wird 1.5 h beschallt, nochmals mit 37 mg KMnO₄ und 74 mg Montmorillonit KSF versetzt und weitere 3 h beschallt. Zur Aufarbeitung werden die organischen Phasen abgetrennt und auf Kieselgel chromatographiert.

### Beispiel 15

### Oxidation von (-)-Isopropyl-2-methyl-4-(2-methylphenyl)-5-oxo-1,4,5,7-tetrahydrofuro[3,4-b]pyridin-3-carboxylat zu Isopropyl-2-methyl-4-(2-methylphenyl)-5-oxo-5,7-dihydrofuro-[3,4-b]pyridin-3-carboxylat mit Mangandioxid

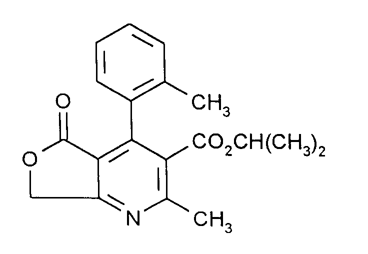

1.1 g (3.36 mmol) (-)-Isopropyl-2-methyl-4-(2-methylphenyl)-5-oxo-1,4,5,7-tetrahydrofuro[3,4-b]pyridin-3-carboxylat werden in 80 ml Chloroform gelöst, mit 5.5 g MnO₂ versetzt und 15 min im Ultraschallbad beschallt. Man saugt über Kieselgur ab und erhält 1 g (91.5 % d. Th.) weiße Kristalle.

### Beispiel 16

### Oxidation von (-)-Isopropyl-2-methyl-4-(2-methylphenyl)-5-oxo-1,4,5,7-tetrahydrofuro[3,4-b]pyridin-3-carboxylat zu Isopropyl-2-methyl-4-(2-methylphenyl)-5-oxo-5,7-dihydrofuro[3,4-b]pyridin-3-carboxylat mit NaNO₂ in Eisessig

50 mg (0.15 mmol) (-)-Isopropyl-2-methyl-4-(2-methylphenyl)-5-oxo-1,4,5,7-tetrahydrofuro[3,4-b]pyridin-3-carboxylat werden in 1.5 ml Eisessig gelöst und innerhalb 20 min mit 50 mg Natriumnitrit versetzt. Anschließend gibt man den Ansatz in eine Lösung von 2.7 g K₂HPO₄ in 5 ml Wasser, schüttelt mit Essigester aus und chromatographiert mit Toluol/Essigester-Gemischen auf Kieselgel.

### Beispiel 17

### Kathodische Reduktion von (+)-Ethyl-2-methyl-4-(2-benzylthiophenyl)-5-oxo-5,7-dihydrofuro[3,4-b]pyridin-3-carboxylat zu (-)-Ethyl-2-methyl-4-(2-benzylthiophenyl)-5-oxo-1,4,5,7-tetrahydrofuro[3,4-b]pyridin-3-carboxylat (S)

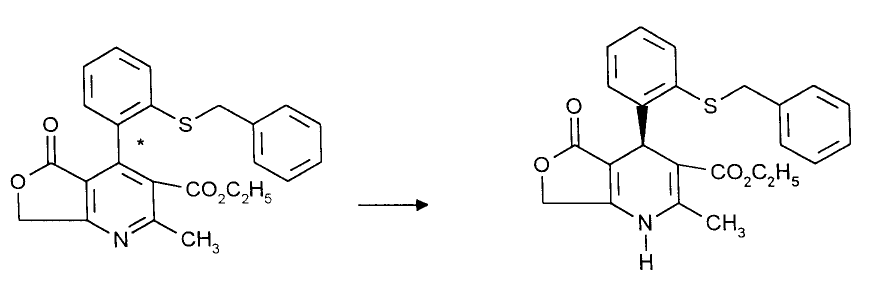

Eine geteilte Zelle mit Hg-Pool-Kathode, Platinanode und Kalomel-Referenzelektrode wird mit einer Lösung aus 120 mg (+)-Ethyl-2-methyl-4-(2-benzylthiophenyl)-5-oxo-5,7-dihydrofuro[3,4-b]pyridin-3-carboxylat und 500 mg TBABF₄ in 50 ml Methanol als Katholyt und ohne Substrat als Anolyt beschickt. Man elektrolysiert stromkontrolliert bei 10 - 20 mA. Die Reaktion wurde nach 2,5 h terminiert, als dünnschichtchromatographisch kein Edukt mehr festzustellen war. Die Methanollösung im Kathodenraum wurde teilweise einrotiert, in Wasser gegeben und der Niederschlag abgesaugt; er wurde in wenig Toluol gelöst auf eine Säule aufgetragen und auf Kieselgel mit Toluol → Toluol / Essigester = 6:1-Gradienten chromatographiert. Die Ausbeute beträgt 45 mg Ethyl-2-methyl-4-(2-benzylthiophenyl)-5-oxo-1,4,5,7-tetrahydrofuro[3,4-b]pyridin-3-carboxylat (38 % d. Th.) mit einem Enantiomerenverhältnis von 90.2 : 9.8.
- R_{f} (SiO₂, Toluol / Essigester = 1 : 1):: (-)-Ethyl-2-methyl-4-(2-benzylthio-phenyl)-5-oxo-1,4,5,7-tetrahydrofuro[3,4-b]pyridin-3-carboxylat: 0,45
chir. HPLC: ee = 80,4 % (Enantiomer 1 in der Elutionsreihenfolge)

### Beispiel 18

### Kathodische Reduktion von (-)-Isopropyl-(2-methoxyethyl)-4-(2-chloro-3-cyanophenyl)-2,6-dimethylpyridin-3,5-dicarboxylat zu Isopropyl-(2-methoxyethyl)-4-(2-chloro-3-cyanophenyl)-2,6-dimethyl-1,4-dihydropyridin-3,5-dicarboxylat

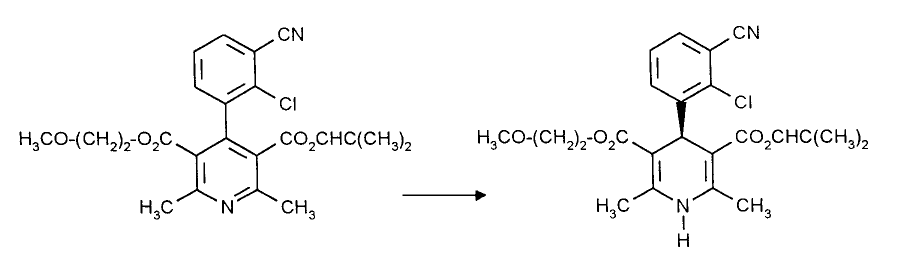

Man verfährt, wie für Beispiel 17 beschrieben. Ausgehend von 120 mg (-)-Isopropyl-(2-methoxyethyl)-4-(2-chloro-3-cyanophenyl-2,6-dimethylpyridin-3,5-dicarboxylat (Atropisomerenverhältnis in der Reihenfolge der Elution von der chiralen HPLC 98 : 2; [α]^{**20**}_{**D**} = -7.2 (c = 0.9, MeOH)) erhält man nach Elektrolyse bei 10-40 mA 80 mg (67 % d. Th.) Isopropyl-(2-methoxyethyl)-4-(2-chloro-3-cyanophenyl)-2,6-dimethyl-1,4-dihydropyridin-3,5-dicarboxylat im Enantiomerenverhältnis 61.3 : 38.7.

### Beispiel 19

### Kathodische Reduktion von Isopropyl-2-methyl-4-(2-methylphenyl)-5-oxo-5,7-dihydrofuro[3,4-b]pyridin-3-carboxylat zu Isopropyl-2-methyl-4-(2-methylphenyl)-5-oxo-1,4,5,7-tetrahydrofuro[3,4-b]pyridin-3-carboxylat

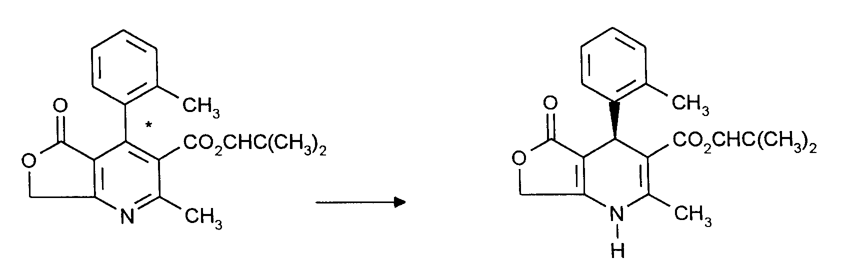

Man verfährt, wie für Beispiel 17 beschrieben. Der Strom beträgt 30 mA für 1.5 h.

Die nachfolgende Tabelle gibt die in den Beispielen 1-19 erzielten Enantiomerenausbeuten wieder:

| **Beispiel-Nr.** | **Enantiomerenverhältnis** | **ee %** ***** |
|---|---|---|
| 1 | 4 : 96 | 92 |
| 2 | 4 : 96 | 92 |
| 3 | 29,5 : 70,5 | 51 |
| 4 | 23 : 77 | 54 |
| 5 | 76,2 : 23,8 | 52,4 |
| 6 | 95:5 (NO₂BF₄); 97,2 : 2,8 (NOBF₄) | 90,6 / 94,4 |
| 7 | 94,3 : 5,7 | 88,6 |
| 8 | 6 : 94 | 88 |
| 9 | 4 : 6 (Dioxan); 48 : 52 (CHCl₃) | 20 / 4 |
| 10 | 70,4 : 29,6 | 64,9 |
| 11 | 59 : 41 | 18 |
| 12 | 45 : 55 | 9,8 |
| 13 | 35 : 65 | 30 |
| 14 | 9 : 91 (Vers. 1); 25,9 : 74,1 (Vers. 2) | 83 / 48 |
| 15 | 16 : 48 | 69 |
| 16 | 3 : 2 | 20 |
| 17 | 90,2 : 9,8 | 85,6 |
| 18 | 61,3 : 38,7 | 23 |
| 19 | 46 : 54 | 11,8 |

| | | |
|---|---|---|
| * Unter Berücksichtigung des ee des Startmaterials wurde der ee für die Reaktion berechnet | | |

## Patentansprüche

1. Verfahren zur Herstellung von optisch aktiven 1,4-Dihydropyridinen, deren chirales C-Atom in 4-Position einen ortho-substituierten Arylrest trägt der allgemeinen Formel (I) 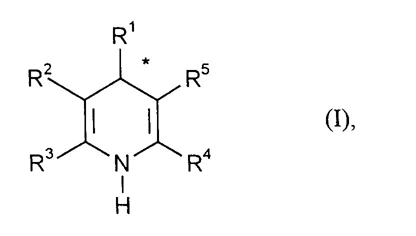 in welcher
R¹ für einen Arylrest der Formel 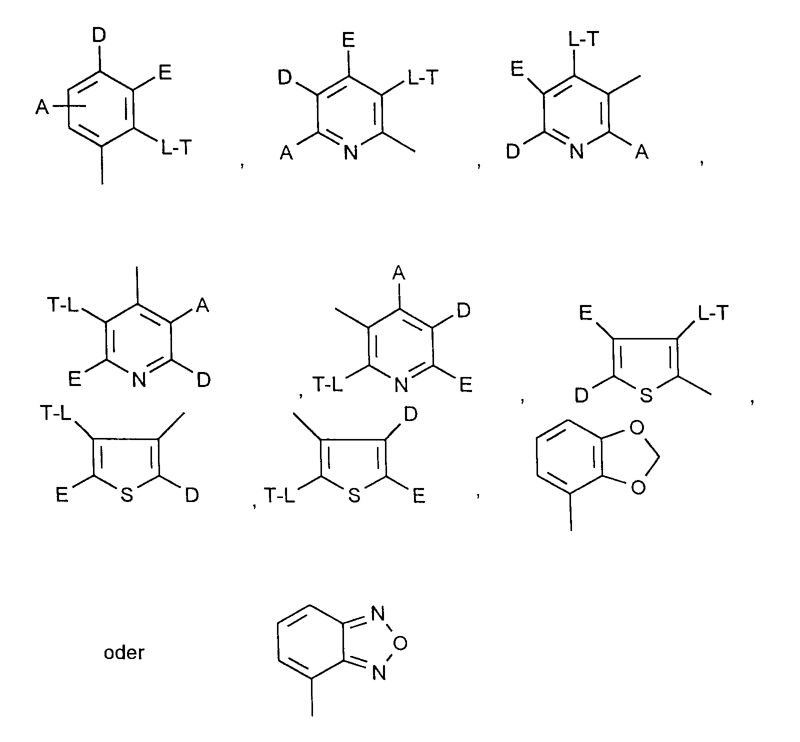 steht,
worin
A, D und E gleich oder verschieden sind und
Wasserstoff, Halogen, Cyano, Trifluormethyl oder für geradkettiges oder verzweigtes Alkyl oder Alkenyl mit jeweils bis zu 6 Kohlenstoffatomen bedeuten, die gegebenenfalls durch Aryl mit 6 bis 10 Kohlenstoffatomen, durch einen 5- bis 7-gliedrigen gesättigten oder ungesättigten Heterocyclus mit bis zu 3 Heteroatomen aus der Reihe S, N und/oder O oder durch geradkettiges oder verzweigtes Alkoxycarbonyl mit bis zu 8 Kohlenstoffatomen oder Carboxyl substituiert sind, oder
eine Gruppe der Formel -NR⁶R⁷, -OR⁸, -S(O)ₐR⁹, -SR¹⁰ oder -P(O)(OR¹¹)(OR¹²) bedeuten,
worin
R⁶, R⁷, R⁸ und R¹⁰ gleich oder verschieden sind und
Wasserstoff, geradkettiges oder verzweigtes Alkyl mit bis zu 8 Kohlenstoffatomen, Benzyl, Aryl mit 6 bis 10 Kohlenstoffatomen oder einen 5- bis 7-gliedrigen gesättigten oder ungesättigten Heterocyclus mit bis zu 3 Heteroatomen aus der Reihe S, N und/oder O bedeuten,
a eine Zahl 1 oder 2 bedeutet,
R⁹ geradkettiges oder verzweigtes Alkyl mit bis zu 6 Kohlenstoffatomen, Benzyl, Phenyl oder Tolyl bedeutet,
R¹¹ und R¹² gleich oder verschieden sind und
Wasserstoff oder geradkettiges oder verzweigtes Alkyl mit bis zu 6 Kohlenstoffatomen bedeuten,
L eine direkte Bindung, ein Schwefel- oder Sauerstoffatom oder einen Rest der Formel -NH oder SO₂ bedeutet,
T in Abhängigkeit von der Definition des Substituenten L eine der chemisch sinnvollen oben angegebenen Bedeutungen von A, D und/oder E hat und mit dieser gleich oder verschieden ist, aber in den Fällen L = Bindung oder -SO₂ nicht für Wasserstoff steht, oder
einen Rest der Formel -CHF₂ bedeutet,
oder
D und E im direkt benachbarten Fall oder E und T unter Einbezug der aro-matischen Doppelbindung jeweils gemeinsam einen 5- bis 8-gliedrigen, partiell ungesättigte oder ungesättigten Carbocyclus oder Heterocyclus mit bis zu 3 Heteroatomen aus der Reihe S, N und/oder O bilden, wobei die Ringsysteme gegebenenfalls bis zu 2-fach gleich oder verschieden durch Halogen, Hydroxy, Nitro, Cyano, Trifluormethyl, durch geradkettiges oder verzweigtes Alkyl oder Alkoxy mit jeweils bis zu 6 Kohlenstoffatomen, durch Aryl mit 6 bis 10 Kohlenstoffatomen oder durch einen 5- bis 6-gliedriegen aromatischen Heterocyclus mit bis zu 3 Heteroatomen aus der Reihe S, N und/oder O substituiert sind,
R² und R⁵ gleich oder verschieden sind und
für Cyano, Nitro, Carboxyl oder für geradkettiges oder verzweigtes Alkoxycarbonyl mit bis zu 6 Kohlenstoffatomen stehen, das gegebenenfalls durch geradkettiges oder verzweigtes Alkoxy oder Acyl mit jeweils bis zu 8 Kohlenstoffatomen oder durch geradkettiges oder verzweigtes Alkyl mit bis zu 6 Kohlenstoffatomen substituiert ist, das seinerseits durch geradkettiges oder verzweigtes Acyl mit bis zu 4 Kohlenstoffatomen, Pyridyl, Furanyl oder durch eine Gruppe der Formel -NR¹³R¹⁴ substituiert sein kann,
worin
R¹³ und R¹⁴ gleich oder verschieden sind und
Wasserstoff, geradkettiges oder verzweigtes Alkyl mit bis zu 6 Kohlenstoffatomen, Aryl mit 6 bis 10 Kohlenstoffatomen oder Benzyl bedeuten, wobei die Ringsysteme gegebenenfalls durch Halogen substituiert sind,
oder
Alkoxycarbonyl gegebenenfalls durch eine Gruppe der Formel -NR¹⁵R¹⁶ substituiert ist,
worin
R¹⁵ und R¹⁶ die oben aufgeführte Bedeutung von R¹³ und R¹⁴ haben und mit dieser gleich oder verschieden sind,
oder
R² und/oder R⁵ für einen Rest der Formel -PO(OR¹⁷)(OR¹⁸), -SO₂ R¹⁹ oder -CO-NR²⁰R²¹ stehen,
worin
R¹⁷ und R¹⁸ die oben angegebene Bedeutung von R¹⁰ und R¹¹ haben und mit diesen gleich oder verschieden sind,
oder
R¹⁷ und R¹⁸ gemeinsam einen Rest der Formel 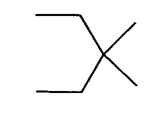 bilden,
R¹⁹ die oben angegebene Bedeutung von R⁹ hat und mit dieser gleich oder verschieden ist,
R²⁰ und R²¹ gleich oder verschieden sind und
Wasserstoff geradkettiges oder verzweigtes Alkyl mit bis zu 6 Kohlenstoffatomen oder Phenyl bedeuten,
R³ und R⁴ gleich oder verschieden sind und
für Amino, Cyano oder für geradkettiges oder verzweigtes Alkinyl oder Alkyl mit jeweils bis zu 4 Kohlenstoffatomen stehen, das gegebenenfalls durch Hydroxy oder durch eine Gruppe der Formel -(O-CO)_{b}-NR²²R²³ substituiert ist,
worin
b eine Zahl 0 oder 1 bedeutet,
R²² und R²³ gleich oder verschieden sind und die oben angegebene Bedeutung von R⁶ und R⁷ haben,
oder
Alkyl gegebenenfalls durch geradkettiges oder verzweigtes Alkoxy mit bis zu 6 Kohlenstoffatomen substituiert ist, das seinerseits durch eine Gruppe der Formel -NR²⁴R²⁵ substituiert sein kann,
worin
R²⁴ und R²⁵ die oben angegebene Bedeutung von R¹³ und R¹⁴ haben und mit dieser gleich oder verschieden sind,
oder
R² und R³ oder R⁴ und R⁵ gemeinsam einen Rest der Formel 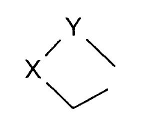 bilden,
worin
X ein Sauerstoff- oder ein Schwefelatom oder eine Gruppe der Formel -NR²⁶ oder -(CH₂)_{c} bedeutet,
worin
R²⁶ Wasserstoff, Phenyl oder geradkettiges oder verzweigtes Alkyl mit bis zu 4 Kohlenstoffatomen bedeutet,
c eine Zahl 1, 2 oder 3 bedeutet,
und
Y eine Gruppe der Formel CO-, SO₂- oder -SO bedeutet,
dadurch gekennzeichnet, daß man entsprechende Dihydropyridine die in 4-Position ein chirales C-Atom besitzen und durch einen ortho-substituierten Aryl- oder Heteroarylrest substituiert sind, die in der synperiplanaren (sp) Form der Formel (Ia) oder antiperiplanaren (ap) Form der Formel (Ib) bzw. deren Spiegelbildern vorliegen 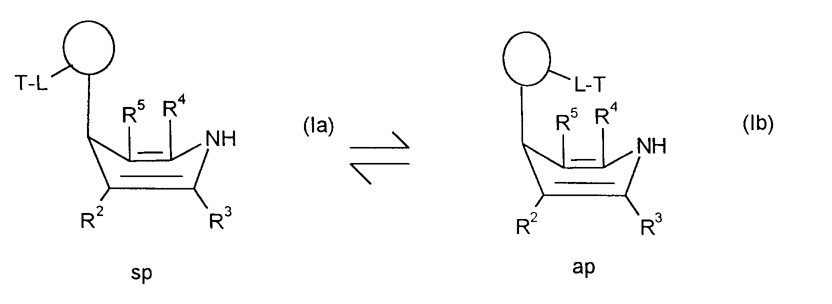 in welchen
R², R³, R⁴, R⁵, L und T die oben angegebene Bedeutung haben
und 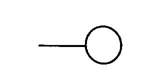 die oben unter dem Substituenten R¹ aufgeführten ortho-substituierten aromatischen Ringsysteme umfaßt,
zunächst durch Oxidation in die atropisomeren Pyridine der allgemeinen Formeln (IIa) bzw. (IIb) 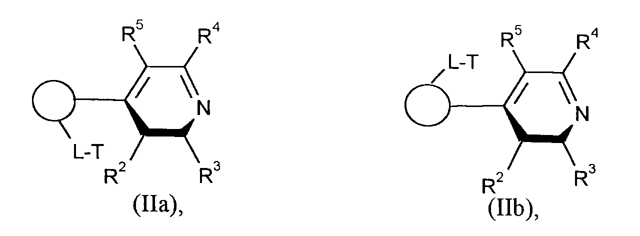 in welchen
R², R³, R⁴, R⁵, L, T und 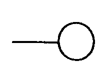 die oben angegebene Bedeutung haben,
überführt und in einem zweiten Schritt diese Pyridine wieder zu 1,4-Dihydropyridinen der Formel (I) reduziert, wobei entweder eine Inversion zum Spiegelbildisomeren oder unter teilweiser Retention eine Racemisierung in unterschiedlichem Ausmaß stattfindet.

2. Verfahren gemäß Anspruch 1, dadurch gekennzeichnet, daß man Dihydropyridine der allgemeinen Formel (I) gemäß Anspruch 1, in welcher
R¹ für einen Rest der Formel 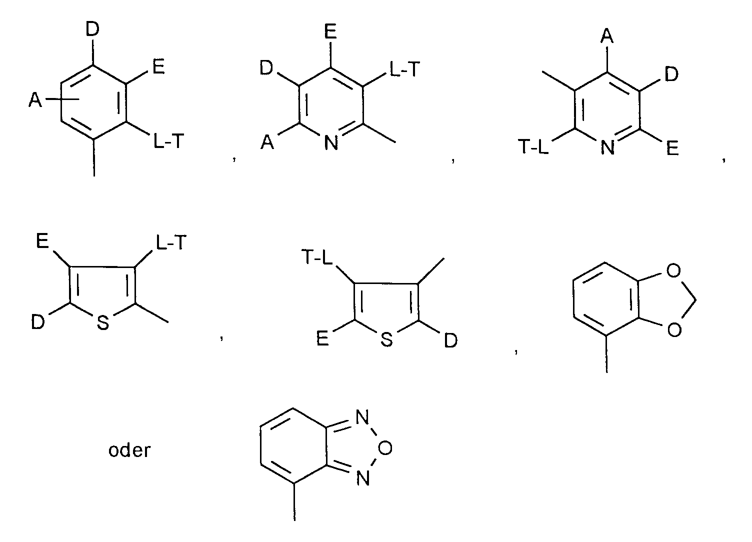 steht,
worin
A, D und E gleich oder verschieden sind und
Wasserstoff, Fluor, Chlor, Brom, Cyano, Trifluormethyl oder für geradkettiges oder verzweigtes Alkyl oder Alkenyl mit jeweils bis zu 5 Kohlenstoffatomen bedeuten, die gegebenenfalls durch Phenyl, Naphthyl, Pyridyl oder durch geradkettiges oder verzweigtes Alkoxycarbonyl mit bis zu 6 Kohlenstoffatomen oder Carboxyl substituiert sind, oder
eine Gruppe der Formel -NR⁶R⁷, -OR⁸, -S(O)ₐR⁹, -SR¹⁰ oder -P(O)(OR¹¹)(OR¹²) bedeuten,
worin
R⁶, R⁷, R⁸ und R¹⁰ gleich oder verschieden sind und
Wasserstoff, geradkettiges oder verzweigtes Alkyl mit bis zu 6 Kohlenstoffatomen, Benzyl Phenyl, Pyridyl, Furyl oder Thienyl bedeuten,
a eine Zahl 1 oder 2 bedeutet,
R⁹ geradkettiges oder verzweigtes Alkyl mit bis zu 4 Kohlenstoffatomen, Benzyl, Phenyl oder Tolyl bedeutet,
R¹¹ und R¹² gleich oder verschieden sind und
Wasserstoff oder geradkettiges oder verzweigtes Alkyl mit bis zu 3 Kohlenstoffatomen bedeuten,
L eine direkte Bindung, ein Schwefel- oder Sauerstoffatom oder einen Rest der Formel -NH oder SO₂ bedeutet,
T in Abhängigkeit von der Definition des Substituenten L eine der chemisch sinnvollen oben angegebenen Bedeutungen von A, D und/oder E hat und mit dieser gleich oder verschieden ist, aber in den Fällen L = Bindung oder -SO₂ nicht für Wasserstoff steht, oder
einen Rest der Formel -CHF₂ bedeutet,
oder
D und E im direkt benachbarten Fall oder E und T unter Einbezug der aromatischen Doppelbindung jeweils gemeinsam einen ankondensierten Cyclopentenyl-, Cyclohexenyl-, Cycloheptenyl, Cycloheptadienyl-, Cyclohexadienyl-, Cycloheptadienyl- oder Pyridylring bilden, wobei die Ringsysteme gegebenenfalls durch Phenyl, Thienyl, geradkettiges oder verzweigtes Alkyl mit bis zu 4 Kohlenstoffatomen oder Pyridyl substituiert sind,
R² und R⁵ gleich oder verschieden sind und für Cyano, Nitro, Carboxyl oder für geradkettiges oder verzweigtes Alkoxycarbonyl mit bis zu 5 Kohlenstoffatomen stehen, das gegebenenfalls durch geradkettiges oder verzweigtes Alkoxy oder Acyl mit jeweils bis zu 6 Kohlenstoffatomen oder durch geradkettiges oder verzweigtes Alkyl mit bis zu 5 Kohlenstoffatomen substituiert ist, das seinerseits durch geradkettiges oder verzweigtes Acyl mit bis zu 3 Kohlenstoffatomen, Pyridyl, Furanyl oder durch eine Gruppe der Formel -NR¹³R¹⁴ substituiert sein kann,
worin
R¹³ und R¹⁴ gleich oder verschieden sind und Wasserstoff, geradkettiges oder verzweigtes Alkyl mit bis zu 5 Kohlenstoffatomen, Phenyl, Naphthyl oder Benzyl bedeuten, wobei die Ringsysteme gegebenenfalls durch Fluor, Chlor oder Brom substituiert sind,
oder
Alkoxycarbonyl gegebenenfalls durch eine Gruppe der Formel -NR¹⁵R¹⁶ substituiert ist,
worin
R¹⁵ und R¹⁶ die oben aufgeführte Bedeutung von R¹³ und R¹⁴ haben und mit dieser gleich oder verschieden sind,
oder
R² und/oder R⁵ für einen Rest der Formel -PO(OR¹⁷)(OR¹⁸), -SO₂ R¹⁹ oder -CO-NR²⁰R²¹ stehen,
worin
R¹⁷ und R¹⁸ die oben angegebene Bedeutung von R¹⁰ und R¹¹ haben und mit diesen gleich oder verschieden sind,
oder
R¹⁷ und R¹⁸ gemeinsam einen Rest der Formel 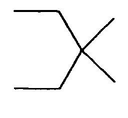 bilden,
R¹⁹ die oben angegebene Bedeutung von R⁹ hat und mit dieser gleich oder verschieden ist,
R²⁰ und R²¹ gleich oder verschieden sind und Wasserstoff, geradkettiges oder verzweigtes Alkyl mit bis zu 4 Kohlenstoffatomen oder Phenyl bedeuten,
R³ und R⁴ gleich oder verschieden sind und für Amino, Cyano oder für geradkettiges oder verzweigtes Alkinyl oder Alkyl mit jeweils bis zu 3 Kohlenstoffatomen stehen, das gegebenenfalls durch Hydroxy oder durch eine Gruppe der Formel -(O-CO)_{b}-NR²²R²³ substituiert ist,
worin
b eine Zahl 0 oder 1 bedeutet,
R²² und R²³ gleich oder verschieden sind und die oben angegebene Bedeutung von R⁶ und R⁷ haben,
oder
Alkyl gegebenenfalls durch geradkettiges oder verzweigtes Alkoxy mit bis zu 4 Kohlenstoffatomen substituiert ist, das seinerseits durch eine Gruppe der Formel -NR²⁴R²⁵ substituiert sein kann,
worin
R²⁴ und R²⁵ die oben angegebene Bedeutung von R¹³ und R¹⁴ haben und mit dieser gleich oder verschieden sind,
oder R² und R³ oder R⁴ und R⁵ gemeinsam einen Rest der Formel 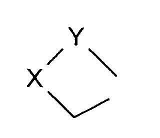 bilden,
worin
X ein Sauerstoff- oder ein Schwefelatom oder eine Gruppe der Formel -NR²⁶ oder -(CH₂)_{c} bedeutet,
worin
R²⁶ Wasserstoff, Phenyl oder geradkettiges oder verzweigtes Alkyl mit bis zu 3 Kohlenstoffatomen bedeutet,
c eine Zahl 1, 2 oder 3 bedeutet,
und
Y eine Gruppe der Formel CO-, SO₂- oder -SO bedeutet,
herstellt.

3. Verfahren zur Herstellung von Verbindungen der allgemeinen Formel (I) gemäß Anspruch 1, in welcher
R¹ für einen Rest der Formel 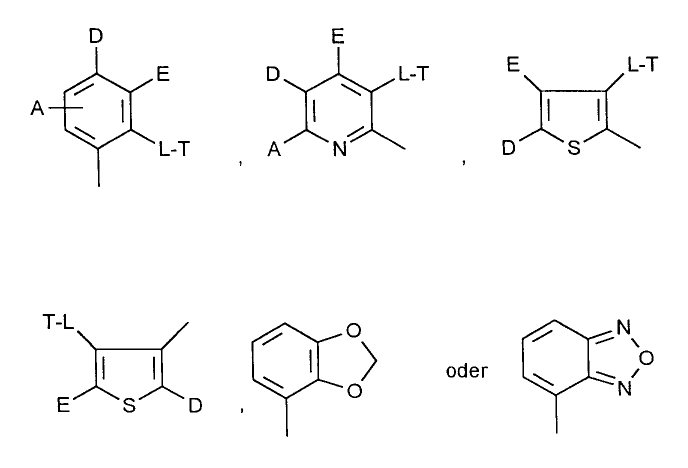 steht,
worin
A, D und E gleich oder verschieden sind und
Wasserstoff, Fluor, Chlor, Brom, Cyano, Trifluormethyl oder geradkettiges oder verzweigtes Alkyl oder Alkenyl mit jeweils bis zu 4 Kohlenstoffatomen bedeuten, die gegebenenfalls durch Phenyl, Naphthyl, Pyridyl oder durch geradkettiges oder verzweigtes Alkoxycarbonyl mit bis zu 5 Kohlenstoffatomen oder Carboxyl substituiert sind, oder
eine Gruppe der Formel -NR⁶R⁷, -OR⁸, S(O)ₐR⁹, -SR¹⁰ oder -P(O)(OR¹¹)(OR¹²) bedeuten,
worin
R⁶, R⁷, R⁸ oder R¹⁰ gleich oder verschieden sind und
Wasserstoff geradkettiges oder verzweigtes Alkyl mit bis zu 4 Kohlenstoffatomen, Benzyl, Phenyl, Pyridyl oder Furyl bedeuten,
a eine Zahl 1 oder 2 bedeutet,
R⁹ Methyl, Benzyl, Phenyl oder Tolyl bedeutet,
R¹¹ und R¹² gleich oder verschieden sind und
Wasserstoff oder geradkettiges oder verzweigtes Alkyl mit bis zu 3 Kohlenstoffatomen bedeuten,
L eine direkte Bindung, ein Schwefel- oder Sauerstoffatom oder einen Rest der Formel -NH oder SO₂ bedeutet,
T in Abhängigkeit von der Definition des Substituenten L eine der chemisch sinnvollen oben angegebenen Bedeutungen von A, D und/oder E hat und mit dieser gleich oder verschieden ist, aber in den Fällen L = Bindung oder -SO₂ nicht für Wasserstoff steht, oder
einen Rest der Formel -CHF₂ bedeutet,
oder
D und E im direkt benachbarten Fall oder E und T unter Einbezug der aromatischen Doppelbindung jeweils gemeinsam einen ankondensierten Cyclopentenyl-, Cyclohexenyl-, Cycloheptenyl, Cyclohexadienyl-, Cycloheptadienyl- oder Pyridylring bilden, wobei die Ringsysteme gegebenenfalls durch Phenyl, Thienyl, geradkettiges oder verzweigtes Alkyl mit bis zu 3 Kohlenstoffatomen oder Pyridyl substituiert sind,
R² und R⁵ gleich oder verschieden sind und Cyano, Nitro oder Carboxyl oder geradkettiges oder verzweigtes Alkoxycarbonyl mit bis zu 4 Kohlenstoffatomen bedeuten, das gegebenenfalls durch geradkettiges oder verzweigtes Alkoxy oder Acyl mit jeweils bis zu 3 Kohlenstoffatomen oder durch geradkettiges oder verzweigtes Alkyl mit bis zu 3 Kohlenstoffatomen substituiert ist, das seinerseits durch geradkettiges oder verzweigtes Acyl mit bis zu 3 Kohlenstoffatomen, Pyridyl, Furanyl oder durch eine Gruppe der Formel -NR¹³R¹⁴ substituiert sein kann,
worin
R¹³ und R¹⁴ gleich oder verschieden sind und Wasserstoff oder geradkettiges oder verzweigtes Alkyl mit bis zu 4 Kohlenstoffatomen bedeuten,
oder
R² und/oder R⁵ für einen Rest der Formel -PO(OR¹⁷)(OR¹⁸), -SO₂ R¹⁹ oder -CO-NR²⁰R²¹ stehen,
worin
R¹⁷ und R¹⁸ die oben angegebene Bedeutung von R¹⁰ und R¹¹ haben und mit diesen gleich oder verschieden sind,
oder
R¹⁷ und R¹⁸ gemeinsam einen Rest der Formel 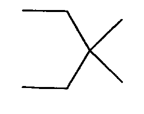 bilden,
R¹⁹ die oben angegebene Bedeutung von R⁹ hat und mit dieser gleich oder verschieden ist,
R²⁰ und R²¹ gleich oder verschieden sind und Wasserstoff oder geradkettiges oder verzweigtes Alkyl mit bis zu 3 Kohlenstoffatomen bedeuten,
R³ und R⁴ gleich oder verschieden sind und für Amino, Cyano oder für geradkettiges oder verzweigtes Alkinyl oder Alkyl mit jeweils bis zu 3 Kohlenstoffatomen stehen, das gegebenenfalls durch Hydroxy oder durch eine Gruppe der Formel -(O-CO)_{b}-NR²²R²³ substituiert ist,
worin
b eine Zahl 0 oder 1 bedeutet,
R²² und R²³ gleich oder verschieden sind und die oben angegebene Bedeutung von R⁶ und R⁷ haben,
oder
Alkyl gegebenenfalls durch geradkettiges oder verzweigtes Alkoxy mit bis zu 3 Kohlenstoffatomen substituiert ist, das seinerseits durch eine Gruppe der Formel -NR²⁴R²⁵ substituiert sein kann,
worin
R²⁴ und R²⁵ die oben angegebene Bedeutung von R¹³ und R¹⁴ haben und mit dieser gleich oder verschieden sind,
oder
R² und R³ oder R⁴ und R⁵ gemeinsam einen Rest der Formel 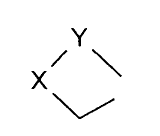 bilden,
worin
X ein Sauerstoff- oder ein Schwefelatom oder eine Gruppe der Formel -NR²⁶ oder -(CH₂)_{c} bedeutet,
worin
R²⁶ Wasserstoff, Phenyl oder geradkettiges oder verzweigtes Alkyl mit bis zu 3 Kohlenstoffatomen bedeutet,
c eine Zahl 1, 2 oder 3 bedeutet,
und
Y die CO-Gruppe bedeutet.

4. Verfahren gemäß Anspruch 1, dadurch gekennzeichnet, daß man als Oxidationsmittel anodische Oxidationen oder NO, HNO₃, NOBF₄ oder NO₂BF₄ einsetzt.

5. Verfahren gemäß Anspruch 1, dadurch gekennzeichnet, daß man die Reduktion durch NaBH₄, BH₃, LiAlH₄ oder Natriumdithionit durchführt oder durch eine kathodische Reduktion in einer übliche Elektrolysezelle, bestehend aus Kathode und Anode und einer Referenzelektrode in Lösung durchführt.

6. Verfahren gemäß Anspruch 1, dadurch gekennzeichnet, daß man als Kathodenmaterial Graphit, Kohle, Pt, Pd, Pb, Cd, Ni oder eine Quecksilberpoolelektrode einsetzt und eine Anode, die aus Kohle, Graphit oder Pd besteht, einsetzt.

7. Verfahren gemäß Anspruch 1, dadurch gekennzeichnet, daß man die kathodische Reduktion spannungskontrolliert zwischen -0,5 V und -2,5 V, oder stromkontrolliert mit Stromstärken von 5 mA bis 1 A durchführt.
